# FASCICULE DE BREVET EUROPEEN

(11) **EP 3 405 598 B1**
(45) Date de publication et mention de la délivrance du brevet: **07.08.2019**
(21) Numéro de dépôt: 17701652.4
(22) Date de dépôt: 16.01.2017
(51) Int. Cl.: C11D 11/00, C23C 16/455, C23F 1/12, C23C 16/44, C23G 5/00, H01J 37/32, C07C 45/00, C23C 14/56

(54) **PROCEDE D'ELIMINATION D'UN DEPOT METALLIQUE DISPOSE SUR UNE SURFACE DANS UNE ENCEINTE**
VERFAHREN ZUR ENTFERNUNG EINES AUF EINER OBERFLÄCHE IN EINER KAMMER ANGEORDNETEN METALLÜBERZUGS
METHOD FOR REMOVING A METAL DEPOSIT ARRANGED ON A SURFACE IN A CHAMBER

(30) Priorité: 19.01.2016 FR 1650407
(43) Date de publication de la demande: 28.11.2018
(73) Titulaire: Kobus SAS, 38330 Montbonnot-Saint-Martin (FR)
(72) Inventeur: VITIELLO, Julien, 38100 Grenoble (FR); PIALLAT, Fabien, 38330 Montbonnot-Saint-Martin (FR)
(74) Mandataire: IP Trust
(86) Numéro de dépôt international: PCT/EP2017/050760
(87) Numéro de publication internationale: WO 2017/125335

(56) Documents cités:
- EP-A1- 1 052 689
- JP-A- 2011 190 490
- US-A1- 2001 009 154

## Description

### DOMAINE DE L'INVENTION

La présente invention concerne un procédé d'élimination d'un dépôt métallique disposé sur une surface dans une enceinte.

### ARRIERE PLAN DE L'INVENTION

Un procédé d'élimination d'un dépôt métallique sur une surface d'une enceinte, connu de l'état de la technique, comprend les étapes suivantes :
a) une étape d'oxydation du dépôt métallique ;
b) une étape d'injection d'espèces chimiques adaptées pour volatiliser le dépôt métallique oxydé, ladite étape b) étant mise en oeuvre pendant au moins une partie de l'étape a).

Cependant, l'efficacité d'un tel procédé d'élimination reste à améliorer.

Ainsi, les espèces chimiques adaptées pour volatiliser le dépôt métallique oxydé peuvent également réagir avec le dépôt métallique avant l'oxydation de ce dernier. L'étape a) s'en trouve bloquée.

Ainsi, la réaction des espèces chimiques avec le dépôt métallique parasite le procédé d'élimination, et surtout nuit à son efficacité.

C'est notamment le cas lorsque le procédé d'élimination est exécuté pour retirer un dépôt de cuivre (Cu) sur une surface dans une enceinte. L'étape a) comprend généralement l'introduction d'oxygène gazeux ou d'ozone gazeux. Les espèces chimiques adaptées pour volatiliser l'oxyde de cuivre comprennent de l'hexafluoroacetylacetone (hfacH). L'espèce chimique hfacH réagit également avec le cuivre avant qu'il ne soit oxydé lorsqu'elle est injectée dans l'enceinte. La réaction d'oxydation est alors bloquée.

L'objet de la présente invention est alors de proposer un procédé d'élimination d'un dépôt métallique permettant de limiter les réactions parasites susceptibles de bloquer ledit procédé.

Un procédé connu de l'art antérieur propose l'élimination d'un dépôt métallique disposé sur une surface dans une enceinte, ledit procédé comprenant la mise en oeuvre répétée d'une séquence comprenant : a) une première phase d'injection d'espèces chimiques adaptées pour oxyder ledit dépôt métallique (2) ; b) une seconde phase d'injection d'espèces chimiques adaptées pour volatiliser le dépôt métallique oxydé, ladite seconde phase b) étant mise en oeuvre après la première phase a).

Une première application de l'invention concerne le nettoyage de résidus métalliques disposés sur les parois intérieures d'une chambre de dépôt.

Une autre application de l'invention concerne la fabrication des circuits imprimés, et plus particulièrement la gravure de couches métalliques utilisées notamment pour le remplissage de vias, qui sont des trous métallisés permettant la connexion électrique entre plusieurs couches d'un circuit imprimé.

De manière classique, les vias sont remplis en excès avec un métal tel que le cuivre, de manière à assurer un remplissage satisfaisant des vias. Le métal en excès est retiré par une étape de gravure chimico-mécanique. Une couche d'arrêt est disposée entre le substrat du circuit imprimé et la couche de dépôt métallique, pour contrôler l'épaisseur de la gravure. L'étape de gravure chimico-mécanique nécessite l'utilisation de couches d'arrêt pour assurer un contrôle très précis du procédé et requiert en outre des opérations ultérieures de nettoyage de la surface gravée, ce qui est complexe et coûteux.

Un autre objet de l'invention est de proposer un procédé de fabrication de vias métallisés simplifié et moins coûteux.

### BREVE DESCRIPTION DE L'INVENTION

La présente invention vise à remédier en tout ou partie aux inconvénients précités et concerne un procédé d'élimination d'un dépôt métallique disposé sur une surface dans une enceinte, ledit procédé comprenant la mise en oeuvre répétée d'une séquence telle que décrite dans la revendication 1 et comprenant :
a) une première phase d'injection d'espèces chimiques adaptées pour oxyder ledit dépôt métallique ;
b) une seconde phase d'injection d'espèces chimiques adaptées pour volatiliser le dépôt métallique oxydé, ladite seconde phase b) étant mise en oeuvre après la première phase a).

Ainsi, l'injection des espèces chimiques adaptées pour volatiliser le dépôt métallique oxydé après l'injection des espèces chimiques adaptées pour oxyder le dépôt métallique permet d'éviter d'une part une réaction desdites espèces entre elles et d'autre part une réaction parasite comprenant la réaction du dépôt métallique disposé sur une surface dans une enceinte avec lesdites espèces chimiques.

Par ailleurs, l'injection, sous la forme d'une ou plusieurs impulsions, des espèces chimiques adaptées pour oxyder le dépôt métallique et pour volatiliser le dépôt métallique oxydé permet également de limiter la quantité desdites espèces chimiques.

Selon l'invention, lors de la phase b), les espèces chimiques sont injectées en quantité sous-stoechiométrique par rapport à la quantité du dépôt métallique oxydé lors de la phase a).

Ainsi, il est possible de limiter la consommation des espèces chimiques adaptées pour volatiliser le dépôt métallique oxydé, et par conséquent de limiter le coût de l'élimination du dépôt.

Selon une forme d'exécution, la séquence comprend une phase entre la phase a) et la phase b) pendant laquelle aucune espèce chimique n'est injectée dans l'enceinte.

Par ailleurs, la séquence peut comprendre une phase de purge au moins partielle de l'enceinte entre la phase a) et la phase b).

D'autre part, le procédé peut comprendre, entre deux séquences consécutives, une étape dans laquelle aucune espèce chimique n'est injectée dans l'enceinte et/ou une purge au moins partielle de l'enceinte est réalisée.

Selon un mode de mise en oeuvre, l'enceinte est maintenue à une température comprise entre 20 et 250°C, de préférence entre 20 et 150°C.

Selon un mode de mise en oeuvre, l'étape a) est exécutée par injection d'une espèce oxydante comprenant au moins une des espèces suivantes : oxygène, ozone, protoxyde d'azote.

Selon un mode de mise en oeuvre, les espèces chimiques injectées à l'étape b) comprennent de l'hexafluoroacetylacetone.

De manière particulièrement avantageuse, les espèces chimiques injectées pendant la phase a) et/ou pendant la phase b) peuvent être activées par plasma.

Selon un mode de mise en oeuvre, le dépôt métallique comprend au moins un des éléments suivants : Cuivre, Titane, Tantale, Ruthénium, Zinc, Zirconium, Vanadium, Argent, Or, Chrome.

Un autre objet de l'invention concerne un procédé de nettoyage de résidus métalliques disposés sur les parois intérieures d'une chambre de dépôt, dans lequel on élimine lesdits résidus au moyen du procédé d'élimination tel que décrit ci-dessus.

Un autre objet de l'invention concerne un procédé de gravure d'un dépôt métallique déposé en excès sur une surface, dans lequel on élimine au moins une partie dudit dépôt métallique au moyen du procédé d'élimination tel que décrit ci-dessus.

Selon un mode de réalisation, préalablement à la première séquence de phases a) et b), un masque est apposé de manière localisée sur le dépôt métallique.

Le procédé de gravure peut alors comprendre les étapes suivantes :
- le masque est apposé sur chaque région du dépôt métallique à conserver ;
- la séquence de phases a) et b) est mise en oeuvre à une ou plusieurs reprises pour éliminer l'excès du dépôt métallique dans chaque région non recouverte par le masque.

De manière alternative, le procédé de gravure peut comprendre les étapes suivantes :
- le masque est apposé sur chaque région du dépôt métallique à graver ;
- des espèces chimiques adaptées pour passiver le dépôt métallique dans chaque région non recouverte par le masque sont injectées ;
- le masque est retiré ;
- la séquence des phases a) et b) est mise en oeuvre à une ou plusieurs reprises pour éliminer l'excès du dépôt métallique dans chaque région préalablement recouverte par le masque.

La séquence de phases a) et b) peut être répétée autant de fois que nécessaire pour éliminer l'excès du dépôt métallique.

### BREVE DESCRIPTION DES FIGURES

L'invention sera mieux comprise à la lumière de la description qui suit des modes de réalisation particuliers non limitatifs de l'invention en référence aux figures ci-jointes parmi lesquelles :

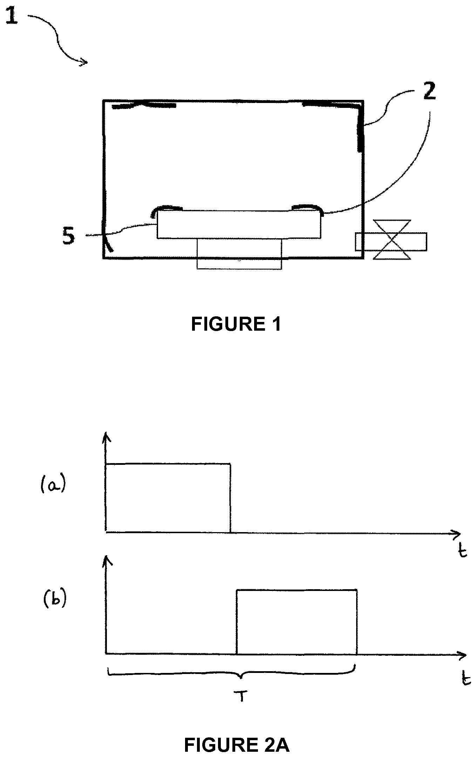

- la figure 1 est une présentation schématique d'une enceinte ;
- la figure 2A représente, sur le graphe (a), la quantité d'espèces oxydantes injectées dans l'enceinte en fonction du temps selon un mode de réalisation de l'invention et, sur le graphe (b), la quantité d'espèces chimiques adaptées pour volatiliser le dépôt métallique oxydé injecté dans l'enceinte en fonction du temps selon ledit mode de réalisation de l'invention, l'échelle de temps étant la même que celle de du graphe (a) ;

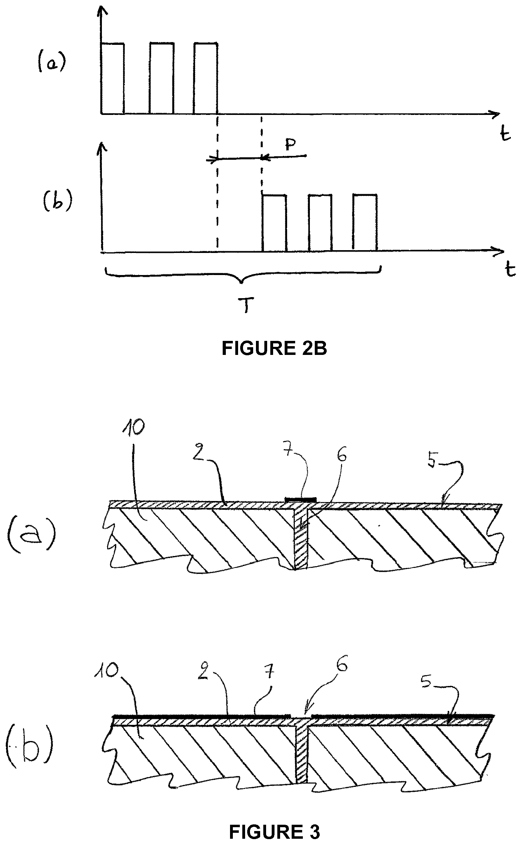

- la figure 2B représente, sur le graphe (a), la quantité d'espèces oxydantes injectées dans l'enceinte en fonction du temps selon un autre mode de réalisation de l'invention et, sur le graphe (b), la quantité d'espèces chimiques adaptées pour volatiliser le dépôt métallique oxydé injecté dans l'enceinte en fonction du temps selon ledit mode de réalisation de l'invention, l'échelle de temps étant la même que celle de du graphe (a) ;
- les figures 3(a) et 3(b) sont des coupes transversales partielles d'un circuit imprimé, le procédé de l'invention étant mis en oeuvre selon deux variantes pour graver un dépôt métallique déposé en excès sur la surface d'un substrat du circuit imprimé.

### DESCRIPTION DETAILLEE DE MODES DE REALISATION DE L'INVENTION

Pour les différents modes de mise en oeuvre, les mêmes références seront utilisées pour des éléments identiques ou assurant la même fonction, par souci de simplification de la description.

La figure 1 représente une enceinte 1 dans laquelle est disposé un support 5.

Un dépôt métallique 2 indésirable est observé sur des surfaces dans l'enceinte 1, à savoir sur les parois intérieures de l'enceinte 1, ainsi que sur le support 5.

Le dépôt métallique 2 peut comprendre au moins un des éléments suivants : Cuivre, Titane, Tantale, Ruthénium, Zinc, Zirconium, Vanadium, Argent, Or, Chrome.

Le procédé d'élimination du dépôt métallique 2 sur les surfaces dans l'enceinte 1 est mis en oeuvre de manière cyclique, par répétition d'une ou plusieurs séquences comprenant chacune une première phase a) consistant à injecter dans l'enceinte des espèces adaptées pour oxyder le dépôt métallique et une seconde phase b) consistant à injecter dans l'enceinte des espèces adaptées pour volatiliser le dépôt métallique oxydé, ladite seconde phase b) étant mise en oeuvre après la fin de la première phase a). En d'autres termes, dans ce procédé, on évite toute injection simultanée des espèces destinées à oxyder le dépôt métallique et des espèces destinées à volatiliser le dépôt métallique oxydé. Les phases a) et b) peuvent éventuellement être séparées par une durée déterminée pendant laquelle aucune espèce n'est injectée dans l'enceinte, ou pendant laquelle une purge au moins partielle de l'enceinte est réalisée,

La phase a) peut être exécutée par injection d'espèces oxydantes sous forme gazeuse par un système d'injection (non représenté).

La phase a) peut être exécutée par injection d'espèces oxydantes comprenant au moins une des espèces suivantes : oxygène, ozone, protoxyde d'azote.

Les espèces oxydantes sont injectées de manière impulsionnelle (sous la forme d'une ou plusieurs impulsions) pendant ladite première phase a) de la séquence de nettoyage.

Suivant des modes de mise en oeuvre :
- la durée de la ou des impulsions d'injection des espèces oxydantes est comprise entre 0,02 s et 5 s, et le délai entre deux impulsions consécutives (le cas échéant) est compris entre 0,02 s et 10 s ;
- la durée de la ou des impulsions d'injection des espèces oxydantes est comprise entre 0,02 s et 1 s, et le délai entre deux impulsions consécutives (le cas échéant) est compris entre 0,02 s et 1 s ;
- la durée de la ou des impulsions d'injection des espèces oxydantes est comprise entre 1 s et 5 s, et le délai entre deux impulsions consécutives (le cas échéant) est compris entre 1 s et 10 s.

Lors de l'injection des espèces oxydantes, une réaction d'oxydation du dépôt métallique 2 se produit en sa surface libre.

Les espèces chimiques adaptées pour volatiliser le dépôt métallique oxydé utilisées dans la deuxième phase de la séquence de nettoyage pourraient également réagir avec le dépôt métallique 2, et ainsi passiver la surface exposée dudit dépôt. Cette réaction de passivation du dépôt métallique 2 est une réaction parasite qui limite ou bloque toute réaction d'oxydation dudit dépôt par les espèces oxydantes.

Afin d'éviter cette réaction parasite, les espèces chimiques adaptées pour volatiliser le dépôt métallique oxydé sont injectées, dans la phase b) après la fin de la phase a), de manière impulsionnelle (sous la forme d'une ou plusieurs impulsions), par un système d'injection (non représenté).

Suivant des modes de mise en oeuvre :
- la durée de la ou des impulsions d'injection des espèces chimiques adaptées pour volatiliser le dépôt métallique oxydé est comprise entre 0,02 s et 5 s, et le délai entre deux impulsions consécutives (le cas échéant) est compris entre 0,02 s et 10 s ;
- la durée de la ou des impulsions d'injection des espèces chimiques adaptées pour volatiliser le dépôt métallique oxydé est comprise entre 0,02 s et 1 s, et le délai entre deux impulsions consécutives (le cas échéant) est compris entre 0,02 s et 1 s ;
- la durée de la ou des impulsions d'injection des espèces chimiques adaptées pour volatiliser le dépôt métallique oxydé est comprise entre 1 s et 5 s, et le délai entre deux impulsions consécutives (le cas échéant) est compris entre 1 s et 10 s.

Bien que les impulsions illustrées sur les figures 2A et 2B présentent une forme de créneau, d'autres formes d'impulsions peuvent être envisagées, dès lors que deux impulsions successives sont séparées par un intervalle de temps pendant lequel aucune espèce chimique adaptée pour oxyder le dépôt métallique ou pour volatiliser le dépôt métallique oxydé n'est injectée.

L'enceinte 1 peut être maintenue à une température comprise entre 20 et 250°C de manière à maintenir sous forme gazeuse les espèces chimiques adaptées pour volatiliser le dépôt métallique. De préférence, la température de l'enceinte 1 est maintenue à une température comprise entre 20 et 150°C, de préférence encore entre 20 et 100°C.

La pression dans l'enceinte 1 est maintenue entre 0,1 et 10 Torr, ou, de manière préférentielle, entre 1 et 5 Torr.

Le procédé débute avantageusement par la phase a) d'une première séquence afin d'oxyder au moins au niveau de sa surface libre le dépôt métallique 2.

Ainsi, une couche de dépôt métallique oxydé recouvre le dépôt métallique 2.

De manière particulièrement avantageuse, la cinétique d'oxydation du dépôt métallique 2 est préalablement déterminée.

La cinétique d'oxydation du dépôt métallique 2 dépend de sa nature et des conditions de sa formation, mais également des conditions d'oxydation de la phase a).

Toutefois, il est à la portée de l'homme du métier de déterminer de manière empirique la cinétique d'oxydation du dépôt métallique 2.

A cet égard, l'homme du métier pourra se référer au document Guangwen Zhou et al., J. Mater. Res., Vol. 20, No. 7 (1684-1694), Jul 2005.

Ainsi, préalablement à la mise en oeuvre du procédé d'élimination du dépôt métallique 2 sur une surface dans l'enceinte 1, il est avantageux de déterminer les cinétiques d'oxydation du dépôt métallique pour différentes conditions d'oxydation, et dresser des abaques pour chacun des types de dépôt métallique 2 susceptibles d'être déposés sur une surface dans l'enceinte 1.

Lesdits abaques peuvent alors être utilisés pour déterminer la quantité du dépôt métallique oxydé pendant une durée prédéterminée et dans des conditions d'oxydation données.

De ce fait, pour la mise en oeuvre du procédé d'élimination du dépôt métallique 2 sur une surface dans l'enceinte 1, l'utilisation desdits abaques permet de déterminer la quantité du dépôt métallique oxydé lors de la phase a) en fonction de la durée de la ou les impulsions et du débit d'espèces injectées au cours de chaque impulsion.

Lors de chaque impulsion de la phase b), la couche de dépôt métallique oxydé est, au moins en partie, volatilisée par les espèces chimiques.

Une fois la première séquence de phases a) et b) achevée, on injecte à nouveau des espèces oxydantes dans l'enceinte (nouvelle phase a)), de sorte que lesdites espèces oxydantes se retrouvent majoritaires dans l'enceinte 1, et par conséquent la réaction d'oxydation du dépôt métallique 2 est la réaction dominante, puis une nouvelle phase b) est mise en oeuvre. Eventuellement, une purge de l'enceinte peut être mise en oeuvre entre la phase b) d'une séquence et la phase a) de la séquence suivante, et/ou un laps de temps pendant lequel aucune espèce n'est injectée dans l'enceinte est respecté entre deux séquences consécutives.

De manière avantageuse, la durée de la ou les impulsions de la phase b) est ajustée de sorte que la couche du dépôt métallique oxydé ne soit pas intégralement volatilisée. Ainsi, la portion de couche du dépôt métallique oxydé restante forme une barrière à la passivation du dépôt métallique 2 par les espèces chimiques injectées lors de la phase b). La réaction de passivation est alors évitée.

Dit autrement, au cours de l'injection des espèces chimiques pour volatiliser le dépôt métallique oxydé dans la phase b), les espèces chimiques sont injectées en quantité sous-stoechiométrique par rapport à la quantité du dépôt métallique oxydé dans la phase a) de la même séquence.

La quantité sous-stoechiométrique susmentionnée est déterminable par la connaissance à la fois de la quantité du dépôt métallique oxydé lors de la phase a), et du mécanisme de réaction de volatilisation dudit dépôt métallique oxydé avec les espèces chimiques.

De manière particulièrement avantageuse, les espèces oxydantes et/ou les espèces destinées à volatiliser le dépôt métallique oxydé sont activées par une source plasma située in situ ou à distance de la chambre. Ainsi, lesdites espèces sont plus réactives et sont donc susceptibles, même si les phases a) et/ou b) sont mises en oeuvre avec de courtes impulsions, d'atteindre toutes les surfaces de l'enceinte recouvertes du dépôt métallique à éliminer.

Le mode d'injection des espèces chimiques adaptées pour oxyder le dépôt métallique et pour volatiliser le dépôt métallique oxydé présente ainsi de nombreux avantages.

Le premier avantage est de rendre le procédé d'élimination selon l'invention efficace. En effet, la réaction parasite comprenant la passivation du dépôt métallique 2 par les espèces chimiques injectées est ainsi neutralisée. La neutralisation de ladite réaction parasite évite d'avoir à ouvrir l'enceinte 1, et d'avoir recours à un processus de décontamination de cette dernière.

Le second avantage est de pouvoir contrôler la quantité d'espèces chimiques injectées lors des phases a) et b), et permet ainsi de réduire le coût du procédé d'élimination.

### Exemples de mise en oeuvre

A titre d'exemple, la phase a) est exécutée par injection d'oxygène selon une impulsion de durée comprise entre 100ms et 1000ms, de préférence entre 100ms et 500ms, par exemple 200ms, avec un débit compris entre 100 et 1000sccm (centimètre cube par minute), de préférence entre 100 et 500sccm, par exemple 300sccm.

Les espèces chimiques adaptées pour volatiliser le dépôt métallique oxydé peuvent comprendre de l'hexafluoroacetylacetone (hfacH).

Les espèces hfacH sont injectées lors de la phase b) selon une impulsion de durée comprise entre 100ms et 1000ms, de préférence entre 100ms et 500ms, par exemple 200ms, avec un débit compris entre 10 et 500mg.min⁻¹ (milligramme par minute), de préférence entre 10 et 100 mg.min⁻¹, par exemple 50 mg.min⁻¹.

La température dans la chambre de dépôt est maintenue à 50 °C.

Ainsi, lorsque le dépôt métallique 2 comprend du cuivre (Cu), les réactions d'oxydation de la phase a) sont les suivantes :

2*Cu* + *O*₂ → 2*CuO*

*4Cu* + *O*₂ → 2*Cu*₂O

Lors de la phase b), l'oxydation de la phase a) cesse, et le dépôt métallique oxydé est volatilisé par les espèces hfacH injectées selon les réactions suivantes :

*2H* + 2hfac*H* + *CuO* → *Cu*(hfac*H*)₂ + *H*₂*O*

*2H* + 2hfac*H* + *Cu*₂*O* → *Cu* + *Cu*(hfac*H*)₂ + *H*₂*O*

La figure 2A illustre un mode de réalisation d'une séquence de phases a) et b), représentant le débit d'espèces injectées dans chacune desdites phases au cours du temps t. Dans ce mode de réalisation, la phase a) consiste en une unique impulsion pour injecter les espèces oxydantes (par exemple un plasma d'oxygène (O₂)). La phase b) suit immédiatement la phase a) et consiste en une unique impulsion pour injecter les espèces destinées à volatiliser le dépôt de cuivre oxydé (par exemple un plasma de hfacH). T indique la durée de la séquence. Dans le cas particulier illustré, la durée de la phase a) est sensiblement égale à la durée de la phase b), et est par exemple de l'ordre de 500 ms. Toutefois, cette valeur, ainsi que la durée relative des phases a) et b), sont purement indicatives et pourront varier selon le débit des espèces injectées et les cinématiques d'oxydation et de volatilisation.

La figure 2B illustre un mode de réalisation d'une séquence de phases a) et b), représentant le débit d'espèces injectées dans chacune desdites phases au cours du temps t. Dans ce mode de réalisation, la phase a) consiste en une série de trois impulsions pour injecter les espèces oxydantes (par exemple un plasma d'oxygène), lesdites impulsions étant séparées par un intervalle de temps dans lequel on fait le vide dans l'enceinte. Un laps de temps est ménagé entre la fin de la dernière impulsion de la phase a) et la première impulsion de la phase b), ce laps de temps, noté P, pouvant éventuellement être mis à profit pour purger l'enceinte. La phase b) consiste en une série de trois impulsions pour injecter les espèces destinées à volatiliser le dépôt de cuivre oxydé (par exemple un plasma de hfacH). T indique la durée de la séquence. Dans le cas particulier illustré, la durée de la phase a) est sensiblement égale à la durée de la phase b), et est par exemple de l'ordre de 1,5 s. Toutefois, ces valeurs, ainsi que la durée relative des phases a) et b) et le nombre d'impulsions effectuées dans chaque phase, sont purement indicatives et pourront varier selon le débit des espèces injectées et les cinématiques d'oxydation et de volatilisation.

Par rapport à un procédé dans lequel on injecterait en continu le plasma d'oxygène et où l'on injecterait de manière impulsionnelle le plasma de hfacH sans interrompre l'injection du plasma d'oxygène, on observe visuellement, avec un procédé selon l'invention, une élimination plus rapide du dépôt de cuivre.

### Applications

La suite de la description présente deux applications dans lesquelles le procédé d'élimination est mis en oeuvre. Les caractéristiques décrites ci-dessus en référence au procédé d'élimination peuvent être transposées à ces deux applications.

Dans une première application de l'invention, le procédé d'élimination est mis en oeuvre pour le nettoyage de résidus métalliques 2 disposés sur les parois intérieures d'une chambre de dépôt 1.

Dans ce premier mode de réalisation, l'enceinte mentionnée dans le procédé d'élimination est une chambre de dépôt 1. La chambre de dépôt 1 peut être une chambre de dépôt chimique en phase vapeur (CVD), dépôt physique en phase vapeur (PVD), dépôt physique en phase vapeur assistée par plasma (PECVD), dépôt par couches atomiques (ALD).

Lors du dépôt d'un film sur un substrat disposé sur le support 5 de la chambre de dépôt 1, des dépôts indésirables du matériau compris dans le film sont observés sur des surfaces dans la chambre de dépôt 1, à savoir sur les parois intérieures de la chambre de dépôt 1, ainsi que sur le support 5.

Au fil des dépôts mis en oeuvre dans la chambre 1, lesdits dépôts indésirables de matériaux s'accumulent sur ces surfaces, et sont une source de contamination importante des films formés sur les substrats.

Les films formés dans ces chambres de dépôt peuvent être des films métalliques, et les dépôts de matériaux compris dans lesdits films observés sur les parois intérieures de la chambre de dépôt 1 sont appelés résidus métalliques 2.

L'invention consiste alors à nettoyer ces résidus métalliques 2 disposés sur les parois intérieures de la chambre de dépôt 1. De manière analogue au procédé d'élimination d'un dépôt métallique décrit ci-dessus, le procédé conforme au premier mode de réalisation comprend la répétition d'une ou plusieurs séquences comprenant chacune :
a) une phase d'injection d'espèces chimiques adaptées pour oxyder les résidus métalliques 2 ;
b) une phase d'injection d'espèces chimiques adaptées pour volatiliser les résidus métalliques oxydés.

La phase b) est mise en oeuvre après la phase a).

Les figures 3(a) et 3(b) montrent deux variantes d'une deuxième application de l'invention, dans laquelle le procédé d'élimination d'un dépôt métallique 2 est mis en oeuvre pour la fabrication d'un circuit imprimé 10, afin de graver un dépôt métallique 2 déposé sur un substrat du circuit imprimé.

Dans l'exemple des figures 3(a) et 3(b), le dépôt métallique 2 est utilisé pour remplir un via 6. Les vias 6 sont des trous métallisés qui permettent la connexion électrique entre une ou plusieurs couches d'un substrat de circuit imprimé.

Aux figures 3(a) et 3(b), un seul via 6 est représenté, étant entendu que le circuit imprimé 10 peut comporter plusieurs vias 6.

Le procédé de l'invention peut également être mis en oeuvre d'une manière plus générale pour graver un dépôt métallique déposé sur la surface d'un substrat. Par exemple, le procédé permet de réaliser un plot sur un substrat de circuit imprimé, à partir d'une couche de dépôt métallique.

Le substrat du circuit imprimé 10 représenté aux figures 3(a) et 3(b) comporte une face 5. Un via 6 traverse au moins partiellement le circuit imprimé 10 pour connecter électriquement la face 5 à une autre couche du circuit imprimé 10, non représentée.

Le dépôt métallique 2 est réalisé en excès. Il remplit le via 6 afin de le métalliser de manière satisfaisante, et recouvre également la face 5 du circuit imprimé 10. Le procédé de l'invention permet de graver cet excès de dépôt métallique 2, de sorte que le dépôt métallique persiste uniquement à l'intérieur du via 6 et dépasse éventuellement à l'extérieur, le long de son axe longitudinal. Autour du via 6 et sur la surface 5, le dépôt métallique 2 est retiré grâce au procédé de l'invention.

Le circuit imprimé 10 est disposé dans une enceinte non représentée, par exemple une chambre de dépôt similaire à la chambre de dépôt 1 décrite en référence à la première application du procédé de l'invention, pour le nettoyage de résidus métalliques.

La figure 3(a) montre une première variante du deuxième mode de réalisation. Préalablement à la mise en oeuvre du procédé, un masque 7 est apposé sur chaque région du dépôt métallique 2 à conserver, au niveau de la surface 5 du circuit imprimé 10. Le masque 7 est centré sur l'axe longitudinal du via 6.

Puis, la séquence de phases a) et b) du procédé est mise en oeuvre à une ou plusieurs reprises conformément à l'invention, pour éliminer l'excès du dépôt métallique 2 dans chaque région de la surface 5 non recouverte par le masque 7.

Lors de la phase a), le dépôt métallique 2 est oxydé uniquement autour du via 6, puisque le masque 7 protège le via 6. Au terme de la phase a), la partie du dépôt métallique 2 située dans le via 6 et le long de son axe longitudinal n'est pas oxydée.

Lors de la phase b), qui est mise en oeuvre après la phase a), des espèces chimiques adaptées pour volatiliser le dépôt métallique 2 oxydé sont injectées dans l'enceinte 1. Lors de la phase b), les espèces chimiques sont injectées selon une séquence d'impulsions.

Les espèces chimiques adaptées pour volatiliser le dépôt métallique 2 oxydé agissent uniquement autour du via 6, le masque 7 protégeant le dépôt métallique 2 au niveau du via 6.

La séquence de phases a) et b) est répétée autant de fois que nécessaire pour retirer progressivement l'épaisseur de la partie excédante du dépôt métallique 2.

La figure 3(b) montre une deuxième variante du deuxième mode de réalisation. Préalablement à la mise en oeuvre du procédé, un masque 7 est apposé sur chaque région du dépôt métallique 2 à graver, au niveau de la surface 5 du circuit imprimé 10 et autour du via 6.

Puis, des espèces chimiques adaptées pour passiver le dépôt métallique 2 dans chaque région non recouverte par le masque sont injectées dans l'enceinte 1. La passivation peut être effectuée en injectant, en excès par rapport à la quantité du dépôt métallique, les espèces oxydantes et/ou les espèces chimiques adaptées pour volatiliser le dépôt métallique 2 oxydé, de préférence en quantité sur-stoechiométrique par rapport à la quantité du dépôt métallique 2.

Ainsi, le dépôt métallique 2 est passivé uniquement au niveau des zones non masquées, c'est-à-dire au niveau du via 6.

Le masque 7 est ensuite retiré.

Puis, la séquence de phases a) et b) du procédé est mise en oeuvre à une ou plusieurs reprises conformément à l'invention, pour éliminer l'excès du dépôt métallique 2 sur la surface 5, dans chaque région préalablement recouverte par le masque.

Lors de la phase a), le dépôt métallique 2 est oxydé uniquement autour du via 6, puisqu'au niveau du via 6, le dépôt métallique est passivé et remplit ainsi une fonction de masque. Au terme de la phase a), le dépôt métallique n'est pas oxydé au niveau du via 6.

Lors de la phase b), qui est mise en oeuvre après la phase a), des espèces chimiques adaptées pour volatiliser le dépôt métallique 2 oxydé sont injectées dans l'enceinte 1. Lors de la phase b), les espèces chimiques sont injectées selon une séquence d'impulsions.

Les espèces chimiques adaptées pour volatiliser le dépôt métallique 2 oxydé agissent uniquement autour du via 6, la passivation protégeant le dépôt métallique 2 au niveau du via 6 à la manière d'un masque.

La séquence de phases a) et b) est répétée autant de fois que nécessaire pour retirer l'excès du dépôt métallique 2.

Par rapport au procédé conventionnel de gravure d'un dépôt métallique par polissage mécano-chimique, l'invention présente les avantages suivants :
- alors que le polissage mécano-chimique requiert le dépôt d'une couche métallique épaisse (typiquement supérieure ou égale à 1,5 µm) pour assurer l'uniformité du polissage ultérieur, la gravure mise en oeuvre dans l'invention permet de déposer uniquement l'épaisseur de métal suffisante au remplissage du via, soit quelques centaines de nanomètres déposés en excès. L'invention permet donc de diminuer d'environ un facteur 10 la quantité de métal à déposer.
- en s'affranchissant du polissage mécano-chimique qui entraîne une contamination importante du substrat, l'invention évite également les étapes de nettoyage consécutives à ce polissage,
- enfin, la formation d'une couche d'arrêt entre le substrat et le dépôt métallique n'est plus nécessaire.

Ainsi, grâce à l'invention, la gravure d'un dépôt métallique déposé sur une surface est simplifiée et moins coûteuse.

Bien entendu l'invention n'est pas limitée aux modes de mise en oeuvre décrits. On peut y apporter des variantes de réalisation sans sortir du cadre de l'invention.

Ainsi, le procédé d'élimination d'un dépôt métallique selon l'invention permet de limiter les réactions parasites susceptibles de bloquer ledit procédé.

### REFERENCES

G. Zhou et al.: Initial oxidation kinetics of Cu(100), (110), and (111) thin films investigated by in situ UHV TEM, J. Mater. Res., Vol. 20, No. 7, Jul 2005

## Revendications

1. Procédé d'élimination d'un dépôt métallique (2) disposé sur une surface (5) dans une enceinte (1), ledit procédé comprenant la mise en oeuvre répétée d'une séquence comprenant :
a) une première phase d'injection d'espèces chimiques adaptées pour oxyder ledit dépôt métallique (2) ;
b) une seconde phase d'injection d'espèces chimiques adaptées pour volatiliser le dépôt métallique oxydé, ladite seconde phase b) étant mise en oeuvre après la première phase a) ;
et dans lequel, lors de la phase b), les espèces chimiques sont injectées en quantité sous-stoechiométrique par rapport à la quantité du dépôt métallique oxydé lors de la phase a), de sorte que la couche du dépôt métallique oxydé ne soit pas intégralement volatilisée.

2. Procédé selon la revendication 1, dans lequel les espèces chimiques adaptées pour oxyder le dépôt métallique (2) et les espèces chimiques adaptées pour volatiliser le dépôt métallique oxydé sont injectées respectivement sous la forme d'une ou plusieurs impulsions.

3. Procédé selon la revendication 2, dans lequel la durée de la ou des impulsions d'injection est comprise entre 0,02 s et 5 s, et le délai entre deux impulsions consécutives le cas échéant est compris entre 0,02 s et 10 s.

4. Procédé selon l'une des revendications précédentes, dans lequel la séquence comprend une phase entre la phase a) et la phase b) pendant laquelle aucune espèce chimique n'est injectée dans l'enceinte.

5. Procédé selon l'une des revendications précédentes, dans lequel la séquence comprend une phase de purge au moins partielle de l'enceinte entre la phase a) et la phase b).

6. Procédé selon l'une des revendications précédentes, comprenant, entre deux séquences consécutives, une étape dans laquelle aucune espèce chimique n'est injectée dans l'enceinte et/ou une purge au moins partielle de l'enceinte est réalisée.

7. Procédé selon l'une des revendications précédentes, dans lequel l'enceinte (1) est maintenue à une température comprise entre 20 et 250°C, de préférence entre 20 et 150°C.

8. Procédé selon l'une des revendications précédentes, dans lequel la phase a) comprend l'injection d'au moins une des espèces oxydantes suivantes : oxygène, ozone, protoxyde d'azote.

9. Procédé selon l'une des revendications précédentes, dans lequel les espèces chimiques injectées pendant la phase b) comprennent de l'hexafluoroacetylacetone.

10. Procédé selon l'une des revendications précédentes, dans lequel les espèces chimiques injectées pendant la phase a) et/ou pendant la phase b) sont activées par plasma.

11. Procédé selon l'une des revendications précédentes, dans lequel le dépôt métallique (2) comprend au moins un des éléments suivants : Cuivre, Titane, Tantale, Ruthénium, Zinc, Zirconium, Vanadium, Argent, Or, Chrome.

12. Procédé de nettoyage de résidus métalliques (2) disposés sur les parois intérieures d'une chambre de dépôt (1), dans lequel on élimine lesdits résidus au moyen du procédé selon l'une des revendications 1 à 11.

13. Procédé de gravure d'un dépôt métallique (2) déposé en excès sur une surface (5), dans lequel on élimine au moins une partie dudit dépôt métallique au moyen du procédé selon l'une des revendications 1 à 11.

14. Procédé selon la revendication 13, **caractérisé en ce que** préalablement à la première séquence de phases a) et b), un masque (7) est apposé de manière localisée sur le dépôt métallique (2).

15. Procédé selon la revendication 14, **caractérisé en ce qu'**il comprend les étapes suivantes :
- le masque (7) est apposé sur chaque région du dépôt métallique (2) à conserver ;
- la séquence de phases a) et b) est mise en oeuvre à une ou plusieurs reprises pour éliminer l'excès du dépôt métallique (2) dans chaque région non recouverte par le masque.

16. Procédé selon la revendication 14, **caractérisé en ce qu'**il comprend les étapes suivantes :
- le masque (7) est apposé sur chaque région du dépôt métallique (2) à graver ;
- des espèces chimiques adaptées pour passiver le dépôt métallique (2) dans chaque région non recouverte par le masque sont injectées ;
- le masque (7) est retiré ;
- la séquence des phases a) et b) est mise en oeuvre à une ou plusieurs reprises pour éliminer l'excès du dépôt métallique (2) dans chaque région préalablement recouverte par le masque.

17. Procédé selon l'une des revendications 13 à 16, **caractérisé en ce que** la séquence de phases a) et b) est répétée autant de fois que nécessaire pour éliminer l'excès du dépôt métallique (2).

## Patentansprüche

1. Verfahren zur Beseitigung einer Metallabscheidung (2) auf einer Oberfläche (5) in einem Behälter (1), wobei besagtes Verfahren die wiederholte Anwendung einer Abfolge bestehend aus folgenden Schritten umfasst:
a) einem ersten Schritt der Einspritzung chemischer Spezies, die für das Oxidieren der Metallscheidung (2) geeignet sind;
b) einem zweiten Schritt der Einspritzung chemischer Spezies, die für die Verflüchtigung der oxidierten Metallabscheidung geeignet sind, wobei besagter zweiter Schritt b) nach dem ersten Schritt b) umgesetzt wird;
und in dem in Schritt b) die chemischen Spezies in Bezug auf die Menge der in Schritt a) oxidierten Metallabscheidungsmenge in unterstöchiometrischer Menge eingespritzt werden, sodass sich die Schicht der oxidierten Metallabscheidung nicht vollständig verflüchtigt.

2. Verfahren nach Anspruch 1, indem die chemischen Spezies, die zur Oxidation der Metallabscheidung (2) und die chemischen Spezies, die zur Verflüchtigung der oxidierten Metallabscheidung geeignet sind, jeweils in Form einer oder mehrerer Impulse eingespritzt werden.

3. Verfahren nach Anspruch 2, indem die Dauer des oder der Einspritzimpulse 0,02 s bis 5 s und die Zeit zwischen gegebenenfalls 2 hintereinander folgenden Impulsen 0,02 s bis 10 s beträgt.

4. Verfahren nach einem der vorhergehenden Ansprüche, indem die Sequenz einen Schritt zwischen Schritt a) und Schritt b) umfasst, in welchem keine chemischen Spezies in die Behälter eingespritzt werden.

5. Verfahren nach einem der vorhergehenden Ansprüche, indem die Abfolge einen Schritt der zumindest teilweisen Entlüftung des Behälters zwischen Schritt a) und Schritt b) umfasst.

6. Verfahren nach einem der vorhergehenden Ansprüche, welches zwischen 2 aufeinander folgenden Abfolgen einen Schritt umfasst, in dem keine chemische Spezies in den Behälter eingespritzt wird und oder zumindest eine Teilentlüftung des Behälters erfolgt.

7. Verfahren nach einem der vorhergehenden Ansprüche, indem die Temperatur des Behälters (1) zwischen 20 und 250°C, vorzugsweise jedoch zwischen 20 und 150°C gehalten wird.

8. Verfahren nach einem der vorhergehenden Ansprüche, indem der Schritt a) die Einspritzung von zumindest einer der folgenden oxidierenden Spezies umfasst: Sauerstoff, Ozon, Stickstoff oder Stickstoffprotoxid.

9. Verfahren nach einem der vorhergehenden Ansprüche, indem die in Schritt b) eingespritzten chemischen Spezies Hexafluoroacetylaceton umfassen.

10. Verfahren nach einem der vorhergehenden Ansprüche, indem die in Schritt a) und/oder in Schritt b) eingespritzten chemischen Spezies durch Plasma aktiviert werden.

11. Verfahren nach einem der vorhergehenden Ansprüche, indem die Metallabscheidung (2) zumindest eines der folgenden Elemente umfasst: Kupfer, Titan, Tantal, Ruthenium, Zink, Zirkonium, Vanadium, Silber, Gold, Chrom.

12. Reinigungsverfahren für metallische Rückstände (2) an den Innenwänden einer Abscheidungskammer (1), bei dem man besagte Rückstände mittels des Verfahrens gemäß einem der Ansprüche 1 bis 11 beseitigt.

13. Ätzverfahren für eine überschüssige Metallabscheidung (2) auf einer Oberfläche (5), in welcher man zumindest einen Teil der Metallabscheidung mittels des Verfahrens gemäß den Ansprüchen 1 bis 11 beseitigt.

14. Verfahren nach Anspruch 13, **dadurch gekennzeichnet, dass** vor der ersten Sequenz der Schritte a) und b) eine Maske (7) lokal auf der Metallabscheidung (2) aufgebracht wird.

15. Verfahren nach Anspruch 14, **dadurch gekennzeichnet, dass** es folgende Schritte umfasst:
- Die Maske (7) wird auf jedem zu erhaltenden Bereich der metallischen Abscheidung (2) aufgebracht;
- Die Abfolge der Schritte a) und b) wird ein oder mehrmals durchgeführt, um die überschüssige Metallabscheidung (2) in jedem nicht von der Maske bedeckten Bereich zu beseitigen.

16. Verfahren nach Anspruch 14, **dadurch gekennzeichnet, dass** es die folgenden Schritte umfasst:
- Die Maske (7) wird auf jeden zu ätzenden Bereich der Metallabscheidung (2) aufgebracht;
- Es werden in jedem, nicht von der Maske abgedeckten Bereich zum Passivieren der Metallabscheidung (2) geeignete chemische Spezies eingespritzt;
- Die Maske (7) wird abgenommen;
- Die Abfolge der Schritte a) und b) wird ein oder mehrmals durchgeführt, um die überschüssige Metallabscheidung (2) in jedem vorab von der Maske bedeckten Bereich zu beseitigen.

17. Verfahren nach einem der Ansprüche 13 bis 16, **dadurch gekennzeichnet, dass** die Abfolge der Schritte a) und b) so oft wiederholt wird, wie dies für die Beseitigung der überschüssigen Metallabscheidung (2) erforderlich ist.

## Claims

1. A method for removing a metal deposit (2) arranged on a surface (5) in a chamber (1), said method including repeatedly performing a sequence including:
a) a first phase of injecting chemical species suitable for oxidizing said metal deposit (2);
b) a second phase of injecting chemical species suitable for volatilizing the oxidized metal deposit, said second phase b) being performed after the first phase a);
and wherein, during the second phase b), the chemical species are injected in sub-stoichiometric quantities relative to the quantity of the metal deposit oxidized during a phase a), so that the layer of oxidized metal deposit is not integrally volatilized.

2. A method according to claim 1, wherein the chemical species suitable for oxidizing the metal deposit (2) and the chemical species suitable for volatilizing the oxidized metal deposit are injected as one or more pulses respectively.

3. A method according to claim 2, wherein the duration of the injection pulse(s) is between 0.02sec and 5sec, and the time between two consecutive pulses, where appropriate, is between 0.02ecs and 10sec.

4. A method according to one of the preceding claims, wherein the sequence comprises a phase between phase a) and phase b) during which no chemical species is injected into the chamber.

5. A method according to one of the preceding claims, wherein the sequence comprises a phase of at least partially draining the chamber between phase a) and phase b) .

6. A method according to one of the preceding claims, comprising, between two consecutive sequences, a step during which no chemical species is injected into the chamber and/or draining of the chamber is at least partially carried out.

7. A method according to one of the preceding claims, wherein the chamber (1) is maintained at a temperature between 20 and 250°C, preferably between 20 and 150°C.

8. A method according to one of the preceding claims, wherein phase a) comprises injecting at least one of the following oxidizing species: oxygen, ozone, nitrous oxide.

9. A method according to one of the preceding claims, wherein the chemical species injected during phase b) comprise hexafluoroacetylacetone.

10. A method according to one of the preceding claims, wherein the chemical species injected during phase a) and/or during phase b) are plasma-activated.

11. A method according to one of the preceding claims, wherein the metal deposit (2) comprises at least one of the following elements: copper, titanium, tantalum, ruthenium, zinc, zirconium, vanadium, silver, gold, chromium.

12. A method for cleaning metal residues (2) disposed on the inner walls of a deposition chamber (1), wherein said residues are removed using the method according to one of claims 1 to 11.

13. A method for etching a metal deposit (2) deposited in excess on a surface (5), wherein at least a portion of said metal deposit is removed using the method according to one of claims 1 to 11.

14. A method according to claim 13, **characterized in that** prior to the first sequence of phases a) and b), a mask (7) is affixed in a localized manner onto the metal deposit (2).

15. A method according to claim 14, **characterized in that** it comprises the following steps:
- the mask (7) is affixed to each region of the metal deposit (2) to be preserved;
- the sequence of phases a) and b) is performed one or several time(s) to remove the excess of metal deposit (2) in each region not covered by the mask.

16. A method according to claim 14, **characterized in that** it comprises the following steps:
- the mask (7) is affixed to each region of the metal deposit (2) to be etched;
- chemical species suitable for passivating the metal deposit (2) in each region not covered by the mask are injected;
- the mask (7) is removed;
- the sequence of phases a) and b) is performed one or several time(s) to remove the excess of metal deposit (2) in each region previously covered by the mask.

17. A method according to one of claims 13 to 16, **characterized in that** the sequence of phases a) and b) is repeated as many times as necessary to remove the excess of metal deposit (2).
